# EUROPEAN PATENT SPECIFICATION

(11) **EP 0 877 632 B1**
(45) Date of publication and mention of the grant of the patent: **19.03.2003**
(21) Application number: 97942946.1
(22) Date of filing: 13.09.1997
(51) Int. Cl.: A61L 24/10, A61M 1/02, C07K 14/75

(54) **PROCESS OF PREPARATION OF AN AUTOLOGOUS FIBRIN GLUE**
VERFAHREN ZUR HERSTELLUNG EINES AUTOLOGEN FIBRINKLEBERS
PROCEDE DE PREPARATION D'UNE COLLE DE FIBRINE AUTOLOGUE

(30) Priority: 18.09.1996 IT MI961914
(43) Date of publication of application: 18.11.1998
(73) Proprietor: Tarantino, Flavio, 07041 Alghero (IT); Benincasa, Carlo, 20092 Cinisello Balsamo (IT); CLAROL A.G., Vaduz (LI)
(72) Inventor: Tarantino, Flavio, 07041 Alghero (IT); Benincasa, Carlo, 20092 Cinisell BALSAMO (IT); IANNINI, ROCCO, 01037 ronciglione (IT); Rao, Walter, 27100 Pavia (IT)
(74) Representative: Marchi, Massimo
(86) International application number: EP9705104
(87) International publication number: WO98011925

(56) References cited:
- EP-A- 0 505 962
- WO-A-86/01814
- WO-A-91/09573
- WO-A-91/16063
- WO-A-96/31245
- US-A- 4 714 457
- F H SILVER ET AL.: "Preparation and use of fibrin glue in surgery" BIOMATERIALS., vol. 16, no. 12, August 1995, GUILDFORD GB, pages 891-903, XP000513088

## Description

The present invention relates to a process of preparation of autologous fibrin glue for surgical use, and more particularly a closed system process, allowing to obtain fibrin glue ready to be used, avoiding submission of blood or its derivatives to the action of external 5 contaminants.

Fibrin glue of bovine or human origin has been known since a long time and successfully used in different surgical conditions. Fibrin is a substance of proteinic nature and originates from the chemical transformation of fibrinogen, a glycoprotide dissolved in blood plasma with a molecular weight around 320,000 D, that during the coagulation process, through the enzyme thrombin, is organized in a stable fibrous structure, namely the fibrin structure.

Glue obtained from fibrin exerts its action through the rapid and durable connection of various biological components, more particularly tissues such as skin, subcutaneous layers, muscular bundles, vessels, prostheses and organs like liver, spleen, kidney, lung. Moreover this glue can be used as a substitute or integration of the surgical sutures and their waterproofing.

The many surgical uses of this glue arise from its main activities such as adhesive, heamostatic, reparative and filling features. In view of its adhesive activity, said glue is being used in:
- Filling of spaces generated by décollement of the sinusal endosteum as said glue together with calcium hydroxy apatite stimulates the regeneration of mature bone, particularly for filling bone cysts;
- treatment of liquoral fistulae;
- eardrum plastics;
- connection of parenchymatous tissue in the operations on kidney, liver, spleen, pancreas, lung;
- glueing vascular ends to each other or to prostheses, more particularly as support and waterproofing of sutures;
- connection of peripheral nerves in microsurgery operations, for instance of the brachial plexus;
- glueing of skin grafts, paradontal edges and others.

Furthermore, in all these operations fibrin glue showed haemostatic activity, diminishing intra-and post operation haemorrhage. Such a property resulted useful above all in operations where haemorrhage is favoured by the existence of cavities, in which haemostasis is difficult, such as post-extraction bone cavities for instance in inclusions, cysts, apicotomies, prostatectomies and post-tonsillectomies.

WO 91/09573 relates to a system for use in the preparation of autologous fibrin sealant and a method including a precipitation step using fibrinogen precipitatiing agents.

US No.4,714,457 relates to a method and apparatus for use in preparation of fibrinogen from a patient's blood including a cryoprecipitation step for precipitating fibrinogen.

The most commonly used fibrin glue is known under the trade name "Tissucol" and is prepared by cryoprecipitation of bovine or human blood. The industrial preparation of said glue is long and gives a commercially available product of rather high cost

As state of the art there is also another fibrin glue obtained by concentration of platelets and derivatives, always starting from bovine or human blood.

This origin of the blood causes the preparation of the known fibrin glues, to fall within the present highly felt problem of the possibility of transmission of known or unknown viral and other diseases.

Therefore in view of the increasing demand, and in some cases the necessity, of products of autologous origin, an object of the present invention is a process of preparation of autologous fibrin glue for surgical use.

The process of autologous preparation of said fibrin glue allows also not to contravene prohibitions and/or beliefs of ethical and/or religious nature, as it does not use patient's blood but only a chemical compound extracted and separated therefrom.

Another object of the invention is a process of production of said fibrin glue which is cheap and allows a rapid preparation of the product that is ready to be used.

Still another object of the present invention is a process of preparation giving the possibility of preparing the basic product of autologous fibrin glue and then freezing said basic product in view of a subsequent use.

A further object of the invention is a process allowing to obtain fibrin glue in the quantity required by the case, avoiding risks of excessive dosage, as it is possible to obtain blood sample in a quantity consistent with the amount of autologous fibrin glue that one has to produce.

Thus the present invention refers to a process of preparation of autologous fibrin glue or surgical use characterized by the fact of comprising the steps of:
making a quantity of blood which is consistent with the amount of fibrin glue needed uncoagulable by adding an anticoagulating agent by thus providing an uncoabulaire blood sample;
b) placing the uncoagulable blood sample in a centrifuge test tube and subjecting it to centrifugation for separating the surnatant, consisting mainly of blood plasma including fibronogen
c) separating the said surnatant by a suitable device provided with a filter;
d) placing the said surnatant in a suitable test tube provided with a filtering membrane and subjecting to centrifugation in a centrifuge adapted to lodge said test tube, thus concentrating fibrinogen in the plasma by separating it from a filtrated residue;
e) subjecting said plasma to freezing for storage and/or to the treatment of the following step f)
f) mixing said plasma as obtained by centrifugation or thawed, with a suitable starter to obtain fibrin glue and with an antifibrinolytic agent thus obtaining said fibrin glue

The features of the process of preparation of autologous fibrin glue according to the invention will be better illustrated in the course of the following detailed description.

Said process refers to the preparation of autologous fibrin glue for surgical use.

Preferably one takes a quantity of venous blood sample of 20 ml, that are made uncoagulable by adding an anticoagulating agent, for instance 1 ml sodiun citrate, or other anticoagulating agents such as EDTA (ethylene diaminotetracetic acid) or heparin.

The so obtained sample is placed in two test tubes of 10 ml each and centrifugated in a conventional laboratory centrifuge at 2,000 rpm for about 5 minutes.

This centrifugation gives a first separation of the blood plasma from the particulate blood portion, mainly comprising erythrocytes.

Then the surnatant is taken fron the test tube through a filtering device, for instance a syringe having a filter where the pore size is preferably between 0.22 µm and 0.45 µm.

In order to obtain a further separation of the blood plasma from the remnant particulate portion, said filtrate placed in a test tube is subjected to a further centrifugation preferably at 2,500 rpm for one hour.

Said test tube is provided with a filtering membrane adapted to filter particles of a size between 80,000 and 300,000 D. Said test tube is placed for said second centrifugation of the above mentioned step d) in a centrifuge for test tubes of 10 ml, but with the head modified so as to lodge test tubes of 50 ml.

The basic product so obtained is essentially constituted by fibrinogen and coagulation factors and can be transformed into fibrin glue preferably within 12 hours.

The basic product, according to a second embodiment, is frozen for subsequent expected use at -18°C and stored for a maximum period of about 12 months. Said product, after being thawed, must be used preferably within 36 hours. The product, either obtained for immediate use or thawed, is mixed with a starter, preferably commercially available thrombin of animal or human origin, for its transformation into fibrin and contemporaneously and/or subsequently with an antifibrinolytic agent, preferably tranexamic acid, for obtaining autologous fibrin glue of suitable concentration.

A third embodiment of the invention consists in taking little quantities of venous blood samples preferably 5 ml, which is rapidly concentrated to obtain little amounts of a very tough preparation ready to be used with the process of the invention.

Although the foregoing embodiments of the process allow to obtain autologous fibrin glue. they require a fair amount of manual operations and are particularly adapted to produce little quantities of fibrin glue for little surgical operations, as they are bound to the use of test tubes, thus samples generally of about 20 ml.

A fourth embodiment of the present invention allows to cany out a process of production of autologous fibrin glue with closed system, so as to produce greater quantities of fibrin glue, sufficient even in case of surgeries of a certain relevance and at the same time reduce manipulations avoiding any exposure of blood or its derivates to the action of external contaminants.

This closed system essentially comprises three bags, namely a first bag or mother bag where the blood that has been taken from the patient is collected, a second bag provided with a horizontal membrane where plasma ultracentrifugation is effected, and a third bag provided with a vertical diaphragm, from which the ingredients contained in the two bag parts are taken at the same time and meet in a terminal delivery member where the preparation of the fibrin glue is carried out at the same moment of its use.

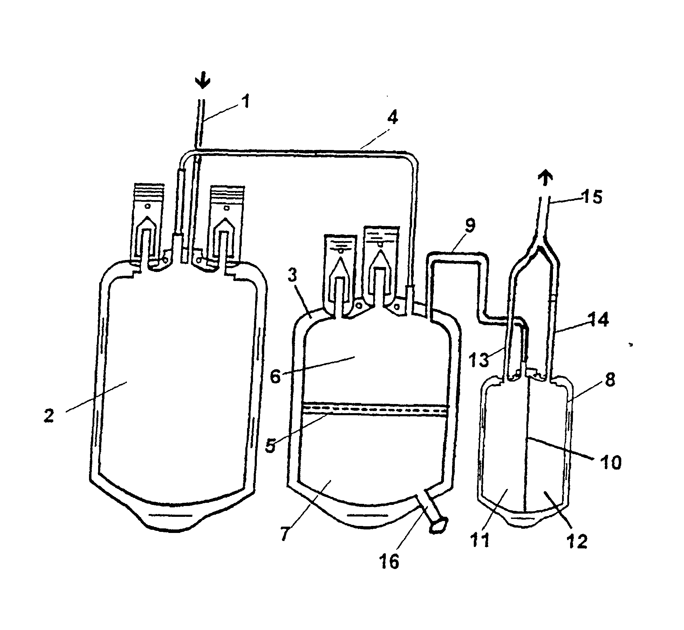

The closed system will now be described in greater detail, with reference to the illustrative diagram shown in the sole figure of the accompanying drawing.

Clearly the bags and the fittings will be made of the most suitable materials and more particularly of anallergic, perfectly sterile plastics of surgical quality.

Moreover bags and fittings may be made in various standard sizes, for treating standardized blood quantities, for instance suitable to receive samples of 50, 100, 200 ml and so forth.

The process according to the fourth embodiment of the invention comprises the following steps, making reference to the illustrative diagram of the attached drawing:
a) A sufficient amount of whole blood sample is fed through a venous inlet and flows into the mother bag 2 where it is mixed with the anticoagulating agent (CPD, ACD and the like), contained in the bag;
b) after having sealed the venous inlet 1 the cell part of the plasma is separated by centrifugation in a centrifuge with oscillating baskets;
c) by means of a bag squeezer the plasma is delivered to the second bag 3, the connection tube 4 is sealed with a bag thermic welder and the mother bag 2 containing concentrated erythrocytes is detached and removed;
d) the bag 3 containing the plasma is provided with a horizontal membrane 5 allowing, after a proper centrifugation period at a suitable rpm, to obtain in the upper part 6 of the bag 3 a fibrinogen enriched plasma while in the lower part 7 the ultrafiltrated residue may be taken through a suitable outlet 16;
e) the fibrinogen enriched plasma is transferred (by gravity in the predetermined quantity) into the third bag 8 and the corresponding connecting tube 9 is sealed with the same technique described at step c). The third bag 8 consists of two parts divided by a vertical diaphragm 10 of which one part 11 (where the fibrinogen enriched plasma was transferred) contains a suitable amount of a substance with antifibrinolytic action such as aprotinin, while the other part 12 contains a suitable amount of a solution of thrombin and calcium chloride;
f) contemporaneous squeezing (manual or through a special device) of the two parts 11, 12 of this last bag 8 allows delivery of the substances contained therein through two plastic connections 13, 14 to a terminal needle 15 allowing the final extemporaneous preparation and use of the fibrin glue ready to be utilized.

It is obviously possible to use, in case it should be necessary, both the concentrated erythrocytes contained in the mother bag 2 and the ultrafiltrate contained in the lower part of the bag 3 provided with the membrane 5.

It is possible to connect the third bag 8 or other dispenser to the bag 3 provided with the membrane 5 through a defluxion member only shortly before the use of the fibrin glue so as to avoid that the dispenser is manipulated during the various working phases.

Finally the third bag 8 or the dispenser may be closed with an external envelope and sterlized separately from the rest of the system so that the absolute sterility also to the outside, can be preserved up to the moment of use.

The quantity of substance with antifibrinolytic action contained in part 11 of the third bag 8, when the substance is aprotinin, is about 3000UIK/ml. The quantity of solution of thrombin and calcium chloride contained in the other part 12 of the third bag 8 is about 50 UI thrombin/ml and 40 µmols CaCl/ml.

It is clear that to the process according to the present invention, and to the relevant closed system all those modifications, additions and substitutions of elements may be resorted to, that might appear to a man skilled in the art, without departing however from its scope, nor falling out from its scope of protection, as defined in the appended claims.

## Claims

1. Process of preparation of autologous fibrin glue for surgical use,
**characterized by** the fact of comprising the steps of:
a) making a quantity of sampled blood, which is consistent with the amount of fibrin glue needed,uncoagulable by adding an anticoagulant agent by thus providing an uncoagulable blood sample;
b) placing the uncoagulable blood sample in a centrifuge test tube and subjecting it to centrifugation for separating the sumatant, consisting mainly of blood plasma including fibrinogen;
c) separating the said surnatant by a suitable device provided with a filter;
d) placing the said surnatant in a suitable test tube provided with a filtering membrane and subjecting to centrifugation in a centrifuge adapted to lodge said test tube, thus concentrating fibrinogen in the plasma by separating it from a filtrated residue;
e) subjecting said plasma to freezing for storage and/or to the treatment of the following step f);
f) mixing said plasma as obtained by centrifugation or thawed, with a suitable starter to obtain fibrin glue and with an antifibrinolytic agent thus obtaining said fibrin glue.

2. Process according to Claim 1, **characterized in that** in said step a) the blood is made uncoagulable by using sodium citrate, EDTA (ethylentetraminoacetic acid) or heparin.

3. Process according to Claim 1, **characterized in that** the said quantity of the blood according to step a) is 20 ml.

4. Process according to Claim 2, **characterized, in that** the said blood is made uncoagulable with a quantity of 1 ml of sodium citrate.

5. Process according to Claim 1, **characterized in that** in said step b) the blood sample is placed in test tubes of 10 ml and subjected to centrifugation at 2000 g for a period of 5 minutes.

6. Process according to Claim 1, **characterized in that** said filtering device of step c) is a syringe with a filter having a pore size between 0.22 µm and 0.45 µm.

7. Process according to Claim 1, **characterized in that** said centrifugation of step d) of the process takes place at 2500 rpm for one hour.

8. Process according to Claim 1, **characterized in that** said test tube for the centrifugation of step d) of the process is a test tube having a filtering membrane filtering particles of size between 80,000 D and 300,000 D.

9. Process according to Claim 1, **characterized in that** the centrifuge for carrying out step d) of the process has a head modified to lodge test tubes of greater size.

10. Process according to Claim 1, **characterized in that** the centrifugated basic product may be frozen at -18°C for a maximum duration of 12 months.

11. Process according to Claim 1, **characterized in that** said starter of step d) of the process is commercially available thrombin of animal or human origin.

12. Process according to Claim 1, **characterized in that** said antifibrinolytic agent of step e) is tranexamic acid or aprotinin.

13. Process according to Claim 1, **characterized in that** the said quantity of blood according to step a) is 5 ml to obtain a very tough preparation for immediate use.

14. Process of production of autologous fibrin glue in a closed system through the use of three different plastic bags connected to one another with sealed tubes, **characterized by** the following steps:
- in the fist bag a blood sample is mixed with an anticoagulant agent , then plasma is separated from erythrocytes by centrifugation after having sealed the venous inlet;
- plasma is transferred to the second bag provided with a horizontal membrane and after having sealed the connecting tube, fibrinogen enriched plasma is separated by ultracentrifugation in the upper part of said second bag while the ultrafiltrate residue remains in the lower part;
- the fibrinogen enriched plasma is transferred to one of two chambers constituting the third bag provided with a vertical diaphragm, where it is mixed with a suitable amount of a substance with antifibrinolytic action, while the other chamber contains a solution of thrombin and calcium chloride, and the connection between said second and third bag is sealed; and
- the contents of the two chambers of said third bag is contemporaneously extracted and meets in a terminal needle. obtaining an extemporaneous preparation of the fibrin glue ready to be used.

15. Process according to Claim 14, **characterized in that** the connecting tubes are sealed by a bag thermic welder.

16. Process according to Claim 14, **characterized in that** the substance with antifibrinolytic action is aprotinin at a concentration of about 3000 UIK/ml.

17. Process according to Claim 14, **characterized in that** the quantity of thrombin 5 contained in the solution of the third bag is about 400 UI/ml and calcium chloride is 40 µmoles/ml.

18. Process according to Claim 14, **characterized in that** the concentrated erythrocytes contained in the first bag and the ultrafiltrate contained in the lower part of the second bag may be used for other working operations.

19. Process according to Claim 14, **characterised in that** the third bag or other dispenser may be connected to the second bag, through a defluxion member, shortly before using the fibrin glue in order to avoid their manipulation during the preceding working phases.

20. Process according to Claim 14, **characterized in that** the third bag or the dispenser are closed with an external envelope and sterilized separately from the rest of the system in 5 order to keep sterility also outside up to the moment of use.

21. Closed system for carrying out the process according to one or more of the preceding claims, **characterized in that** it comprises three bags connected to one another by scalable connecting tubes, namely:
- a first bag or mother bag provided with a venous inlet, where a blood sample is fed and a first centrifugation is carried out to separate plasma from erythrocytes;
- a second bag provided with a horizontal membrane in which the ultracentrifugation of plasma is carried out so as to separate fibrinogen enriched plasma in the upper part of the bag while the ultrafiltrated residue is collected in its lower part; and
- a third bag provided with a vertical diaphragm dividing it in two chambers, in one of which said fibrinogen enriched plasma is transferred and mixed with a substance having antifibrinolytic action, while in the others chamber there is a solution of thrombin and calcium chloride, each chamber of said third bag being provided with a connecting tube meeting in a common terminal dispensing device, so that squeezing contemporaneously the two chambers the contents of the two chambers are mixed and the extemporaneous preparation of the autologous fibrin glue ready to be used is obtained.

22. System according to Claim 21, **characterized in that** the bags and the connections are made of anallergic and perfectly sterile plastics of surgical quality.

23. Use of the closed system according to Claim 21 for the preparation of autologous fibrin glue ready to be used, according to the process recited in one or more of Claims 14-20.

## Patentansprüche

1. Verfahren zum Bereitstellen eines autogenen Fibrinklebstoffs, **dadurch gekennzeichnet, dass** es die Schritte umfasst:
a) Schaffen einer Menge einer Blutprobe, die mit der benötigen Menge von Fibrinklebstoff konsistent ist, unkoagulierbar durch Hinzufügen eines Antikoaguliermittels, wodurch eine unkoagulierbare Blutprobe bereitgestellt wird;
b) Platzieren der unkoagulierbaren Blutprobe in einem Zentrifugentestrohr und Unterwerfen derselben einer Zentrifugation, um das Surnatant zu trennen, das hauptsächlich aus Blutplasma besteht, welches Fibrinogen aufweist;
c) Trennen des Surnatants durch eine geeignete Vorrichtung, die mit einem Filter ausgestattet ist;
d) Platzieren des Surnatants in einem Testrohr, das mit einer Filtermembran ausgestattet ist, und Unterwerfen einer Zentrifugation in einer Zentrifuge, die zum Aufnehmen des Testrohrs ausgelegt ist, wodurch Fibrinogen in dem Plasma durch Trennen desselben von einem gefilterten Rest konzentriert wird;
e) Unterwerfen des Plasmas einem Gefrieren zur Lagerung und/oder zur Behandlung im nachfolgenden Schritt f);
f) Mischen des durch Zentrifugation erhaltenen oder getauten Plasmas mit einem geeigneten Auslöser, um Fibrinklebstoff zu erhalten, und mit einem antifibrinolytischen Mittel, wodurch der Fibrinklebstoff erhalten wird.

2. Verfahren nach Anspruch 1, **dadurch gekennzeichnet, dass** in Schritt a) das Blut mittels Schwefelzitrat, EDTA (Ethylendiamintetraessig-Säure) oder Heparin unkoagulierbar gemacht wird.

3. Verfahren nach Anspruch 1, **dadurch gekennzeichnet, dass** die Menge der Blutprobe gemäß Schritt a) 20 ml ist.

4. Verfahren nach Anspruch 2, **dadurch gekennzeichnet, dass** das Blut mit einer Menge von 1 ml Schwefelzitrat unkoagulierbar gemacht wird.

5. Verfahren nach Anspruch 1, **dadurch gekennzeichnet, dass** in Schritt b) die Blutprobe in Testrohren von 10 ml platziert wird und Zentrifugation bei 2000 g für eine Dauer von 5 Minuten unterworfen wird.

6. Verfahren nach Anspruch 1; **dadurch gekennzeichnet, dass** die Filtervorrichtung aus Schritt c) eine Spritze mit einem Filter ist, der eine Porengröße zwischen 0,22 µm und 0,45 µm besitzt.

7. Verfahren nach Anspruch 1, **dadurch gekennzeichnet, dass** die Zentrifugation aus Schritt d) des Verfahrens bei 2500 U/min für eine Stunde stattfindet.

8. Verfahren nach Anspruch 1, **dadurch gekennzeichnet, dass** das Testrohr für die Zentrifugation aus Schritt d) des Verfahrens ein Testrohr ist, das eine Filtermembran besitzt, die Partikel einer Größe zwischen 80.000 D und 300.000 D filtert.

9. Verfahren nach Anspruch 1, **dadurch gekennzeichnet, dass** die Zentrifuge zum Durchführen von Schritt d) des Verfahrens einen Kopf besitzt, der zum Aufnehmen von Testrohren größerer Abmessung modifiziert ist.

10. Verfahren nach Anspruch 1, **dadurch gekennzeichnet, dass** das zentrifugierte Basisprodukt bei -18° C für eine maximale Dauer von 12 Monaten eingefroren werden kann.

11. Verfahren nach Anspruch 1, **dadurch gekennzeichnet, dass** der Auslöser aus Schritt d) des Verfahrens handelsüblich verfügbares Thrombin tierischen oder menschlichen Ursprunges ist.

12. Verfahren nach Anspruch 1, **dadurch gekennzeichnet, dass** das antifibrinolytische Mittel aus Schritt e) Tranexamsäure oder Aprotinin ist.

13. Verfahren nach Anspruch 1, **dadurch gekennzeichnet, dass** die Blutmenge nach Schritt a) 5 ml ist, um eine sehr robuste Zubereitung für sofortigen Gebrauch zu erhalten.

14. Verfahren zur Herstellung autogenen Fibrinklebstoffs in einem geschlossenen System durch die Verwendung von drei unterschiedlichen Kunststoffbeuteln, die mit abgedichteten Rohren miteinander verbunden sind, **gekennzeichnet durch** die folgenden Schritte:
- in dem ersten Beutel wird eine Blutprobe mit einem Antikoalugationsmittel gemischt, dann wird Plasma von Erythrozyten **durch** Zentrifugation getrennt, nachdem der venöse Einlass abgedichtet worden ist;
- das Plasma wird in den zweiten Beutel übertragen, der mit einer horizontalen Membran ausgestattet ist, und nachdem das Verbindungsrohr abgedichtet worden ist, wird mit Fibrinogen angereichertes Plasma **durch** Ultrazentrifugation in dem oberen Teil des zweiten Beutels getrennt, während der ultrafiltrierte Rest in dem unteren Teil verbleibt;
- das mit Fibrinogen angereicherte Plasma wird zu einer von zwei Kammern übertragen, welche den dritten Beutel bilden, der mit einem vertikalen Diaphragma ausgestattet ist, wo es mit einer geeigneten Menge einer Substanz mit antifibrinolytischer Wirkung gemischt wird, während die andere Kammer eine Lösung aus Thrombin und Kalziumchlorid enthält, und die Verbindung zwischen dem zweiten und dem dritten Beutel wird abgedichtet; und
- die Inhalte der zwei Kammern des dritten Beutels werden gleichzeitig extrahiert und treffen sich in einer Anschlussnadel, wodurch eine sofortige Zubereitung des Fibrinklebstoffs erzielt wird, der zur Verwendung bereit ist.

15. Verfahren nach Anspruch 14, **dadurch gekennzeichnet, dass** die Verbindungsrohre durch einen thermischen Beutelschweißer abgedichtet sind.

16. Verfahren nach Anspruch 14, **dadurch gekennzeichnet, dass** die Substanz mit antifibrinolytischer Wirkung Aprotinin bei einer Konzentration von etwa 3000 UIK/ml ist.

17. Verfahren nach Anspruch 14, **dadurch gekennzeichnet, dass** die Thrombinmenge, die in der Lösung des dritten Beutels enthalten ist, etwa 400 UI/ml ist, und Kalziumchlorid beträgt 40 µmol/ml.

18. Verfahren nach Anspruch 14, **dadurch gekennzeichnet, dass** die konzentrierten Erythrozyten, die in dem ersten Beutel enthalten sind, und das Ultrafiltrat, das in dem unteren Teil des zweiten Beutels enthalten ist, für weitere Arbeitsvorgänge verwendet werden können.

19. Verfahren nach Anspruch 14, **dadurch gekennzeichnet, dass** der dritte Beutel oder andere Spender mit dem zweiten Beutel verbunden sein können, durch ein Ausgusselement, kurz bevor der Fibrinkleber verwendet wird, um deren Beeinträchtigung während der vorhergehenden Arbeitsphasen zu vermeiden.

20. Verfahren nach Anspruch 14, **dadurch gekennzeichnet, dass** der dritte Beutel oder der Spender mit einem äußeren Umschlag verschlossen sind und getrennt von dem Rest des Systems sterilisiert werden, um auch außerhalb des Zeitpunkts der Verwendung die Sterilität aufrechtzuerhalten.

21. Geschlossenes System zum Durchführen des Verfahrens nach einem oder mehreren der vorhergehenden Ansprüche, **dadurch gekennzeichnet, dass** es drei miteinander durch abdichtbare Verbindungsrohre verbundene Beutel aufweist, nämlich:
- einen ersten Beutel oder Mutterbeutel, der mit einem venösen Einlass ausgestattet ist, an dem eine Blutprobe zugeführt wird, und eine erste Zentrifugation wird durchgeführt, um Plasma von Erythrozyten zu trennen;
- ein mit einer horizontalen Membran ausgestatteter zweiter Beutel, in welchem die Ultrazentrifugation des Plasmas derart ausgeführt wird, um mit Fibrinogen angereichertes Plasma in dem oberen Teil des Beutels zu trennen, während der ultrafiltrierte Rest in dessen unteren Teil gesammelt wird; und
- einen dritten Beutel, der mit einem vertikalen Diaphragma ausgestattet ist, welches diesen in zwei Kammern teilt, in einer von denen das mit Fibrinogen angereicherte Plasma übertragen wird und mit einer Substanz gemischt wird, die antrifibrinolytische Wirkung besitzt, während es in der anderen Kammer eine Lösung aus Thrombin und Kalziumchlorid gibt, wobei jede Kammer des dritten Beutels mit einem Verbindungsrohr ausgestattet ist, die sich in einer gemeinsamen Anschlussspendervorrichtung treffen, so dass gleichzeitiges Quetschen der zwei Kammern die Inhalte der zwei Kammern mischt und es wird die sofortige Zubereitung des einsatzbereiten, autogenen Fibrinklebstoffs erhalten.

22. System nach Anspruch 21, **dadurch gekennzeichnet, dass** die Beutel und die Verbindungen aus nicht-allergischen, perfekt sterilen Kunststoffen chirurgischer Qualität hergestellt sind.

23. Verwendung des geschlossenen Systems nach Anspruch 21 zur Zubereitung einsatzbereiten, autogenen Fibrinklebstoffs, gemäß dem Verfahren nach einem oder mehreren der Ansprüche 14 bis 20.

## Revendications

1. Procédé de préparation de colle de fibrine autologue à usage chirurgical, **caractérisé en ce qu'**il comprend les étapes consistant à:
a) rendre une quantité de sang échantillonné, qui est cohérente avec la quantité de colle de fibrine nécessaire, incoagulable en ajoutant un agent anticoagulant et en donnant ainsi un échantillon sanguin incoagulable;
b) placer l'échantillon de sang incoagulable dans une éprouvette de centrifugation et le soumettre à une centrifugation pour séparer le surnageant, principalement constitué de plasma sanguin comprenant du fibrinogène;
c) séparer ledit surnageant à l'aide d'un dispositif adapté muni d'un filtre;
d) placer ledit surnageant dans une éprouvette adaptée munie d'une membrane filtrante et le soumettre à la centrifugation dans une centrifugeuse adaptée pour loger ladite éprouvette, concentrant ainsi le fibrinogène dans le plasma en le séparant d'un résidu filtré;
e) soumettre ledit plasma à une congélation pour le stockage et/ou au traitement de l'étape f) suivante;
f) mélanger ledit plasma tel qu'il a été obtenu par centrifugation, ou décongelé, avec un produit de départ adapté pour obtenir de la colle de fibrine et avec un agent antifibrinolytique, permettant ainsi d'obtenir ladite colle de fibrine.

2. Procédé selon la revendication 1, **caractérisé en ce que**, à ladite étape a), le sang est rendu incoagulable en utilisant du citrate de sodium, de l'EDTA (acide éthylène diamine tétracétique) ou de l'héparine.

3. Procédé selon la revendication 1, **caractérisé en ce que** ladite quantité de sang selon l'étape a) est 20 ml.

4. Procédé selon la revendication 2, **caractérisé en ce que** ledit sang est rendu incoagulable avec une quantité de 1 ml de citrate de sodium.

5. Procédé selon la revendication 1, **caractérisé en ce que**, à ladite étape b), l'échantillon sanguin est placé dans des éprouvettes de 10 ml et soumis à une centrifugation à 2000 t/min pendant une durée de 5 minutes.

6. Procédé selon la revendication 1, **caractérisé en ce que** ledit dispositif de filtration de l'étape c) est une seringue munie d'un filtre ayant une taille de pores entre 0,22 µm et 0,45 µm.

7. Procédé selon la revendication 1, **caractérisé en ce que** ladite centrifugation de l'étape d) du procédé se déroule à 2500 t/min pendant une heure.

8. Procédé selon la revendication 1, **caractérisé en ce que** ladite éprouvette pour la centrifugation de l'étape d) du procédé est une éprouvette ayant une membrane filtrante qui filtre des particules d'une taille comprise entre 80 000 D et 300 000 D.

9. Procédé selon la revendication 1, **caractérisé en ce que** la centrifugeuse pour réaliser l'étape d) du procédé possède une tête modifiée pour recevoir des éprouvettes de plus grande taille.

10. Procédé selon la revendication 1, **caractérisé en ce que** le produit de base centrifugé peut être congelé à -18°C pendant une durée maximum de 12 mois.

11. Procédé selon la revendication 1, **caractérisé en ce que** ledit produit de départ de l'étape d) du procédé est de la thrombine disponible dans le commerce d'originale animale ou humaine.

12. Procédé selon la revendication 1, **caractérisé en ce que** ledit agent antifibrinolytique de l'étape e) est de l'acide tranexamique ou de l'aprotinine.

13. Procédé selon la revendication 1, **caractérisé en ce que** ladite quantité de sang selon l'étape a) est 5 ml, pour obtenir une préparation très résistante pour une utilisation immédiate.

14. Procédé de production de colle à base de fibrine autologue dans un système fermé par l'utilisation de trois poches en plastique différentes reliées les unes aux autres avec des tubes scellés, **caractérisé par** les étapes suivantes :
dans la première poche, un échantillon sanguin est mélangé avec un agent anticoagulant, puis le plasma est séparé des érythrocytes par centrifugation après avoir scellé l'entrée veineuse ;
le plasma est transféré vers la deuxième poche munie d'une membrane horizontale et, après avoir scellé le tube de raccordement, le plasma enrichi en fibrinogène est séparé par ultracentrifugation dans la partie supérieure de ladite deuxième poche pendant que le résidu de l'ultrafiltration reste dans la partie inférieure ;
le plasma enrichi en fibrinogène est transféré vers l'une des deux chambres constituant la troisième poche munie d'une membrane verticale, où il est mélangé avec une quantité adaptée d'une substance à effet antifibrinolytique, tandis que l'autre chambre contient une solution de thrombine et de chlorure de calcium, et le raccordement entre lesdites troisième et quatrième poches est scellé ; et
les contenus des deux chambres de ladite troisième poche sont extraits en même temps et se rencontrent dans une aiguille terminale, donnant une préparation extemporanée de la colle de fibrine prête à être utilisée.

15. Procédé selon la revendication 14, **caractérisé en ce que** les tubes de raccordement sont scellés par un soudeur thermique de poches.

16. Procédé selon la revendication 14, **caractérisé en ce que** la substance à effet antifibrinolytique est de l'aprotinine à une concentration d'environ 3000 UIK/ml.

17. Procédé selon la revendication 14, **caractérisé en ce que** la quantité de thrombine contenue dans la solution de la troisième poche est d'environ 400 UI/ml et la quantité de chlorure de calcium est de 40 µmoles/ml.

18. Procédé selon la revendication 14, **caractérisé en ce que** les érythrocytes concentrés contenus dans la première poche et l'ultrafiltrat contenu dans la partie inférieure de la deuxième poche peuvent être utilisés pour d'autres opérations de travail.

19. Procédé selon la revendication 14, **caractérisé en ce que** la troisième poche, ou autre distributeur, peut être reliée à la deuxième poche, par l'intermédiaire d'un élément de défluxion, peu avant d'utiliser la colle de fibrine afin d'éviter leur manipulation pendant les phases de travail précédentes.

20. Procédé selon la revendication 14, **caractérisé en ce que** la troisième poche ou le distributeur est fermé(e) à l'aide d'une enveloppe externe et stérilisé(e) séparément du reste du système afin de conserver la stérilité également à l'extérieur jusqu'au moment de l'utilisation.

21. Système fermé pour mettre en oeuvre le procédé selon une ou plusieurs des revendications précédentes, **caractérisé en ce qu'**il comprend trois poches reliées les unes aux autres par des tubes de raccordement scellables, à savoir :
une première poche, ou poche principale, munie d'une entrée veineuse, où un échantillon sanguin est amené et une première centrifugation est effectuée pour séparer le plasma des érythrocytes ;
une deuxième poche munie d'une membrane horizontale dans laquelle l'ultracentrifugation du plasma est effectuée afin de séparer le plasma enrichi en fibrinogène dans la partie supérieure de la poche tandis que le résidu ultrafiltré est recueilli dans sa partie inférieure ; et
une troisième poche munie d'une membrane verticale la divisant en deux chambres, dans une desquelles ledit plasma enrichi en fibrinogène est transféré et mélangé à une substance à effet antifibrinolytique, tandis que dans l'autre chambre il y a une solution de thrombine et de chlorure de calcium, chaque chambre de ladite troisième poche étant munie d'un tube de raccordement qui se retrouve dans un dispositif de distribution terminal commun, de telle sorte qu'en comprimant simultanément les deux chambres le contenu des deux chambres soit mélangé et que la préparation extemporanée de la colle de fibrine autologue prête à l'emploi soit obtenue.

22. Système selon la revendication 21, **caractérisé en ce que** les poches et les raccordements sont réalisés dans une matière plastique anallergique et parfaitement stérile de qualité chirurgicale.

23. Utilisation du système fermé selon la revendication 21 pour la préparation de colle de fibrine autologue prête à l'emploi, selon le procédé décrit dans une ou plusieurs des revendications 14 à 20.
